# EUROPEAN PATENT APPLICATION

(11) **EP 4 123 659 A1**
(43) Date of publication of application: **25.01.2023**
(21) Application number: 22185377.3
(22) Date of filing: 18.07.2022
(51) Int. Cl.: G16H 20/40, G16H 50/70

(54) **SURVIVAL DECISION TREE GRAPHS FOR PERSONALIZED TREATMENT PLANNING**

(30) Priority: 19.07.2021 US 202117379183
(71) Applicant: Biosense Webster (Israel) Ltd, Yokneam, 2066717 (IL)
(72) Inventor: TSOREF, Liat, 2066717 Yokneam (IL); AMIT, Matityahu, 2066717 Yokneam (IL); TAL-BOTZER, Ronen, 2066717 Yokneam (IL); ZAHAVI, Guy Sender, 2066717 Yokneam (IL); BEN-AMI, Lee, 2066717 Yokneam (IL)
(74) Representative: Carpmaels & Ransford LLP

(57) **Abstract**

A method includes receiving a data set including medical information of respective patients, respective types of a clinical procedure performed on the patients, and respective survival rates of the patients. A survival tree graph is generated by maximizing a cost function of differences in the survival rates between the types of the clinical treatment procedure. A type of the clinical procedure is selected for a given patient, based on the survival tree graph.

## Description

### FIELD OF THE INVENTION

This invention relates generally to personalized medicine, and specifically to matching cardiac ablation procedures to patients.

### BACKGROUND OF THE INVENTION

Methods of personalized healthcare predictions based on the analysis of health metrics of a population of patients were previously reported in the patent literature. For example, U.S. Patent Application Publication No. 2018/0158552 provides a method for creating an interpretable model for healthcare predictions that includes training, by a deep learning processor, a neural network to predict health information by providing training data, including multiple combinations of measured or observed health metrics and corresponding medical results, to the neural network. The method also includes determining, by the deep learning processor and using the neural network, prediction data including predicted results for the measured or observed health metrics for each of the multiple combinations of the measured or observed health metrics based on the training data. The method also includes training, by the deep learning processor or a learning processor, an interpretable machine learning model to make similar predictions as the neural network by providing mimic data, including combinations of the measured or observed health metrics and corresponding predicted results of the prediction data, to the interpretable machine learning model.

As another example, U.S. Patent Application Publication 2019/0019581 describes methods and apparatus that provide digital diagnostics and digital therapeutics to patients. The digital personalized medicine system uses digital data to assess or diagnose symptoms of a patient, and feedback from the patient response to treatment is considered to update the personalized therapeutic interventions. The methods and apparatus disclosed herein can also diagnose and treat cognitive function of a subject, with fewer questions, decreased amounts of time, and determine a plurality of behavioral, neurological or mental health disorders, and provide clinically acceptable sensitivity and specificity in the diagnosis and treatment.

U.S. Patent Application Publication No. No. 2020/0098451 describes a facility for predicting patient outcomes on the basis of clinical trials. The facility obtains information describing one or more completed clinical trials, and extracts features from the obtained clinical trial information. The facility uses the extracted features to train both a time-series data model for predicting clinical outcomes and a non-time-series data model for predicting clinical outcomes. The facility applies these trained models to information describing a subject patient to predict a clinical outcome for the subject patient.

### SUMMARY OF THE INVENTION

An embodiment of the present invention provides a method including receiving a data set including medical information of respective patients, respective types of a clinical procedure performed on the patients, and respective survival rates of the patients. A survival tree graph is generated by maximizing a cost function of differences in the survival rates between the types of the clinical treatment procedure. A type of the clinical procedure is selected for a given patient, based on the survival tree graph.

In some embodiments, the differences in the survival rates include differences between Kaplan-Meier (KM) curves.

In some embodiments, generating the survival tree graph includes applying a genetic algorithm to the data set.

In other embodiments, applying the genetic algorithm includes selecting decision nodes and cutoff values that maximize homogeneity in each split in the survival tree graph.

In an embodiment, generating the survival tree graph further includes fixing one or more layers of the survival tree graph.

In another embodiment, fixing the layers includes fixing a root of the survival tree graph.

In yet another embodiment, wherein fixing the root of the survival tree graph includes presetting the root to be one of an age and a gender of a patient.

In some embodiments, fixing the layers includes presetting a layer that precedes an output layer of the survival tree graph.

In other embodiments, presetting the layer that precedes the output layer includes presetting the layer to indicate a treatment approach.

In further embodiments, the treatment approach includes pulmonary vein isolation.

In some embodiments, the types of the clinical procedure are one of different cardiac ablation treatments of cardiac arrhythmia, different treatments of cancer, and different of treatments of brain stroke.

There is additionally provided, in accordance with an embodiment of the present invention, a system including an interface and a processor. The interface is configured for receiving a data set including medical information of respective patients, respective types of a clinical procedure performed on the patients, and respective survival rates of the patients. The processor is configured to (i) generate a survival tree graph by maximizing a cost function of differences in the survival rates between the types of the clinical treatment procedure, and (ii) select a type of the clinical procedure for a given patient, based on the survival tree graph.

There is further provided, in accordance with an embodiment of the present invention, a computer software product, the product including a tangible non-transitory computer-readable medium in which program instructions are stored, which instructions, when read by a processor, cause the processor to (i) generate a survival tree graph by maximizing a cost function of differences in the survival rates between the types of the clinical treatment procedure, and (ii) select a type of the clinical procedure for a given patient, based on the survival tree graph.

### BRIEF DESCRIPTION OF THE DRAWINGS

The present disclosure will be more fully understood from the following detailed description of the embodiments thereof, taken together with the drawings, in which:
Fig. 1 is a schematic, pictorial illustration of a catheter-based electrophysiological (EP) sensing, EP signal analysis, and ablation system, according to an exemplary embodiment of the present invention;
Figs. 2 and 3 illustrate survival tree graphs generated by a genetic algorithm using Kaplan-Meier (KM) survival curves, to select an optimal treatment procedure for a given patient, according to exemplary embodiments of the present invention; and
Fig. 4 is a flow chart of a method for generating and using the genetic algorithm of Fig. 2 to select the optimal treatment procedure, according to an exemplary embodiment of the present invention.

### DETAILED DESCRIPTION OF EMBODIMENTS

### OVERVIEW

Survival analysis of a patient population performed over a time period (e.g., months and years) after undergoing a certain medical treatment may indicate a general efficacy of the treatment. However, such an indication is an average that may be inaccurate for a specific given patient. For example, several cardiac ablation treatments of atrial fibrillation (AF) are used by different physicians (e.g., electrophysiologists). Survival analysis may be performed on each of such treatments, amongst them being:
- Pulmonary vein isolation (PVI) only.
- PVI and additional anatomical isolation lines.
- PVI and/or additional ablation that is based on electrophysiological (EP) mapping results. The mapping may indicate particular sources of AF, such as scars.
- Non PVI, usually ablation that is based on non-PV trigger mapping such as CFAE, triggers, rotors, fractionations, etc.

Different studies show the efficacy and performance of the different ablation treatments listed above, but these studies include recruitment of different (e.g., a wide range of) patients, so there are no clear guidelines to which type of patient a respective specific ablation approach may be most beneficial.

Embodiments of the present invention that are described herein provide machine learning (ML) based methods to estimate and recommend the best cardiac ablation procedure for a specific patient, from among a list of predefined cardiac ablation procedures. To this end, an embodiment of the invention includes a method and an algorithm for generating a treatment (e.g., ablation) decision tree graph that maximizes survival chances of a given patient by proper selection of treatment (e.g., ablation). The decision tree graph divides a database of the study population into distinct groups with different survival measures (e.g., different Kaplan-Meier (KM) curves). Using the decision tree graph to differentiate between competing treatment approaches, a clinician can select a best treatment approach (e.g., cardiac ablation) for a specific patient.

In some embodiments, a processor runs a genetic algorithm prepared in accordance with the disclosed technique to find a hierarchy of decision parameters (nodes), and a cost function that is based on a resulting level of distinctiveness between different KM curves (leaves) of a path of the decision tree graph. The nodes may comprise patient age, gender, and clinical information. In another embodiment, a portion of the hierarchy is given to the genetic algorithm, which finds the rest of the survival-tree nodes. For example, in case of cardiac ablation, a possible root of the tree graph may be predetermined to be age, or predetermined to be gender. The one layer before the output layer may contain nodes predetermined to be the different ablation treatment approaches.

Using the disclosed method, a clinician operating a an EP mapping and ablation system can offer a new patient a treatment among the ML-analyzed treatments, e.g., one that best matches the patient's profile and diagnostic data so as to provide a best expected clinical outcome. In case of AF, the disclosed technique leverages available EP information (e.g., an EP map) on the heart of a patent to assist a clinician to determine an optimal cardiac ablation procedure to treat, for example, a severe arrhythmia, among possible approaches, for a specific patient based on survival, as measured by survival rate.

### SYSTEM DESCRIPTION

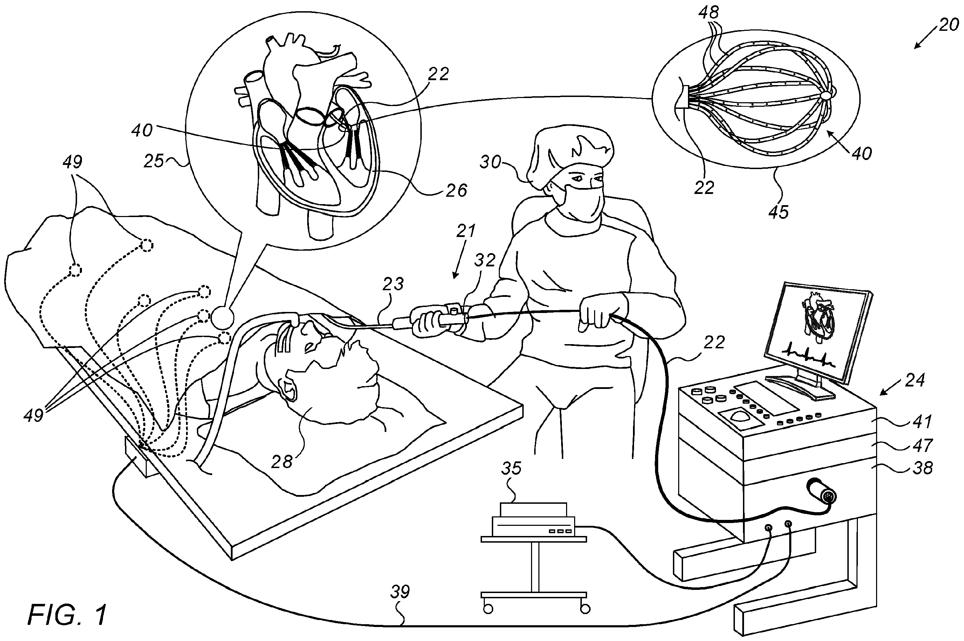

Fig. 1 is a schematic, pictorial illustration of a catheter-based electrophysiological (EP) sensing, EP signal analysis, and ablation system 20, according to an exemplary embodiment of the present invention. System 20 may be, for example, a CARTO^{®} 3 system, produced by Biosense-Webster, Irvine, California. As seen, system 20 comprises a catheter 21, having a shaft 22 that is navigated by a physician 30 into a heart 26 of a patient 28. In the illustrated example, physician 30 inserts shaft 22 through a sheath 23, while manipulating shaft 22 using a manipulator 32 near the proximal end of the catheter 21.

In the exemplary embodiment described herein, catheter 21 may be used for any suitable diagnostic purpose and/or tissue ablation, such as electrophysiological mapping of heart 26 and/or ablation, respectively. An ECG recording instrument 35 may receive various types of ECG signals sensed by system 20 during the process.

As shown in insets 25 and 45, a distal end of shaft 22 of catheter 21 is fitted with a multi-electrode basket catheter 40. Inset 45 shows an arrangement of multiple electrodes 48 of basket catheter 40.

The proximal end of catheter 21 is connected to a control console 24, to transmit, for example, electrograms (EGMs) acquired by electrodes 48. Console 24 comprises a processor 41, typically a general-purpose computer, with suitable front end and interface circuits 38 for receiving EP signals (e.g., EGM and ECG signals) as well as non-EP signals (such as position signals) from electrodes 48 of catheter 21. For this purpose, processor 41 is connected to electrodes 48 via wires running within shaft 22.

Interface circuits 38 are further configured to receive ECG signals, such as from a multichannel (e.g., 12-lead) ECG apparatus that can be ECG recording instrument 35, as well as non-ECG signals from surface body electrodes 49. Typically, electrodes 49 are attached to the skin around the chest and legs of patient 28. Processor 41 is connected to electrodes 49 by wires running through a cable 39 to receive signals from electrodes 49.

During an EP mapping procedure, the locations of electrodes 48 are tracked while they are inside heart 26 of the patient. For that purpose, electrical signals are passed between electrodes 48 and body surface electrodes 49. Based on the signals, and given the known positions of electrodes 22 on the patient's body, a processor 41 calculates an estimated location of each electrode 22 within the patient's heart. Such tracking may be performed using the Advanced Current Location (ACL) system, made by Biosense-Webster (Irvine California), which is described in US Patent No. 8,456,182, whose disclosure is incorporated herein by reference.

The processor 41 may thus associate any given signal received from electrodes 48, such as EGMs, with the location at which the signal was acquired. Processor 41 uses information contained in these signals to construct an EP map, such as a local activation time (LAT) map, to present on a display.

Physician 30 may decide which ablation procedure to perform based on the aforementioned survival decision tree graph inferenced for patient 28 by a processor. To perform ablation, electrodes 48 are connected (e.g., switched) to an ablation waveform generator 47 in console 24. Using guidelines for ablation provided by the disclosed technique, such as type of pulmonary vein isolation to perform, processor 41, or the physician, may select which electrodes to connect to generator 47 to apply ablation.

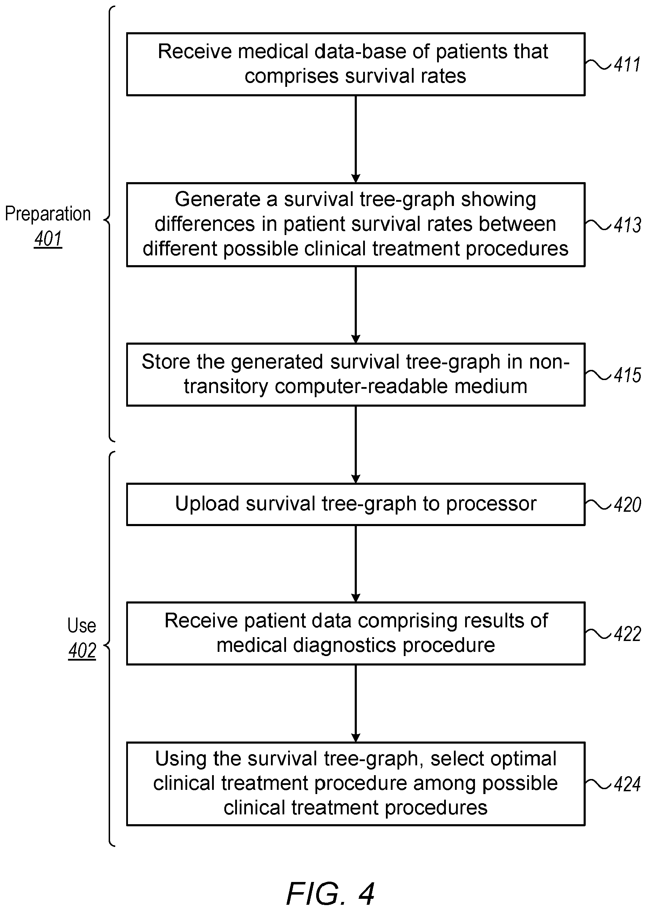

Processor 41 is typically programmed in software to carry out the functions described herein. The software may be downloaded to the processor in electronic form, over a network, for example, or it may, alternatively or additionally, be provided and/or stored on non-transitory tangible media, such as magnetic, optical, or electronic memory. In particular, processor 41 runs a dedicated algorithm as disclosed herein, such as included in Fig. 4, that enables processor 41 to perform the disclosed steps, as further described below.

### SURVIVAL DECISION TREE GRAPHS FOR PERSONALIZED TREATMENT PLANNING

Survival analysis is the phrase used to describe the analysis of data in the form of time durations from a well-defined time origin of a medical treatment of a patient until the occurrence of some particular medical event or endpoint associated with the patient. The treatment may be drug administration (e.g., chemotherapy), or an invasive procedure (e.g., stent placement, ablation) and others.

Various statistical methods have been developed to describe and analyze such time-related outcomes. Among these, Kaplan-Meier curves are widely used in medical research for survival analysis. Kaplan-Meier (KM) curves are an estimation of the survivor function, which is the rate of event occurrence over time. KM curves are described, for example, by Rich JT, et. al, in a paper entitled "A practical guide to understanding Kaplan-Meier curves," Otolaryngology Head Neck Surgery, 143 (3), 2010, pages 331-336.

KM curves can be calculated and drawn for different subgroups of the study-recruited patients. Statistical tests, such as the log-rank test, can be used to compare KM.

A survival decision tree may therefore include:
1. KM curve "leaves" describing treatment failure rates over time
2. Parameters immediately prior to the leaves, for example, the aforementioned different ablation approaches including or not including PVI
3. Additional parameters which comprise a set of decision rules in the upper decision nodes, characterizing subgroups of patients
4. The rules of the tree, which can be created to maximize the difference between each pair of KM curves according to different scores:
   a. Area between the KM curves
   b. Distance between the treatment failure rates at the final time point
   c. The p-value of the log-rank test
   d. Risk concordance index (also called Harrell's c-index)

The disclosed algorithm is aimed at creating an optimal separation between the treatment options (e.g., PVI vs. "PVI+") at the final leaves of the tree graph, using the KM survival curves for the different treatment approaches. To solve these issues, artificial intelligence is used to focus on the final leaves of the tree. The goal is to effectively identify subgroups in which there is a maximal difference between treatments for atrial fibrillation, according to one of the above scores, or norms, (a)-(d). One option for this task is an evolutionary algorithm that is adapted to construct an optimal survival decision tree.

Evolutionary algorithms mimic the biological process of evolution with generations of solutions, selection of the best (fittest) solution, cross-over, mutations, and genetic drifts. Evolutionary algorithms provide a flexible framework to find the optimal survival tree that compares treatment approaches for atrial fibrillation. These are some of their advantages:
1. Massive search of the possible solutions space:
   a. Constructing a survival decision tree involves many considerations, including which features are included in the tree, at what level they are included, what cutoff values are chosen, and others. Since there are many considerations, the number of possible trees is too large to evaluate all possible solutions.
   b. Evolutionary algorithms consider a massive number of solutions in the process of algorithm evolution. Through a process that mimics evolution, the best solutions are evaluated, and an optimal solution can be found.
2. Flexibility in the construction of possible solutions:
   a. Evolutionary algorithms can be fitted to solve problems with different constraints. In the case of survival decision trees, the main constraint is the fixation of the final split of the tree, which might be the atrial fibrillation treatment approach.
   b. Other relevant constraints may be considered as well, including (but not limited to):
      i. The minimal number of subjects in each final Kaplan-Meier curve
      ii. The number of levels of the entire tree
      iii. Fixation of a variable to the first split of the tree, or any other split in the tree

The decision tree is generated by passing data down from a root node to leaves. The data is repeatedly split according to predictor variables so that child nodes are more "pure" (i.e., homogeneous) in terms of the outcome variable. In an embodiment, the disclosed genetic algorithm is configured to select decision nodes and cutoff values that maximize homogeneity in each split in the tree graph.

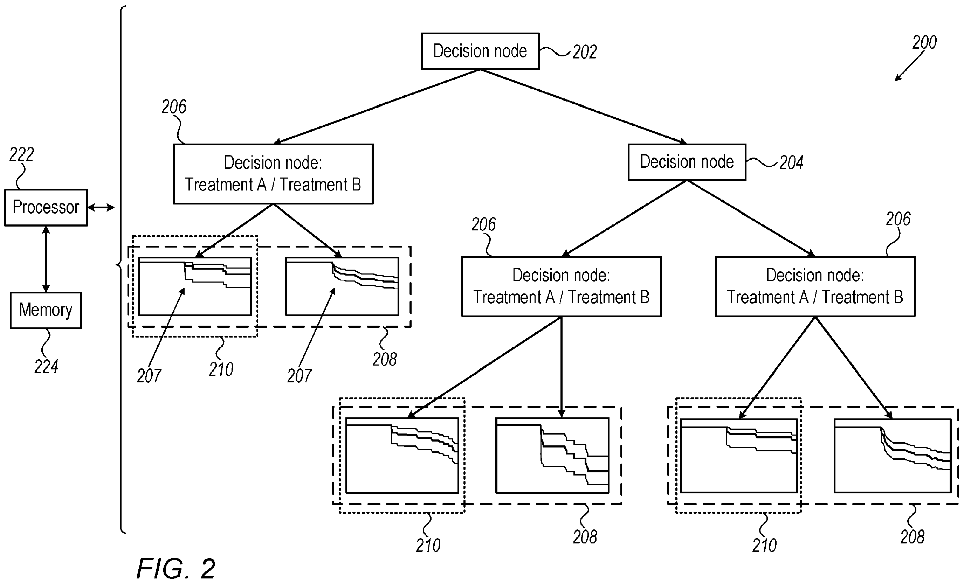

Fig. 2 illustrates a survival tree graph 200 generated by a genetic algorithm using Kaplan-Meier (KM) survival curves, to select an optimal treatment procedure for a given patient according to an embodiment of the present invention. The genetic algorithm that generates decision tree graph 200 is operated by a computer processor 222 using patient data uploaded from a memory 224 that can be local memory or a remote location. In operation, the processor saves the generated tree graph into memory 224.

As seen, survival tree graph 200 comprises a root decision node 202. Tree graph 200 further comprises decision nodes 204 at different layers of the graph. Decision node 202 and 204 may be, for example, one of age, gender, tumor size in case of cancer, or identity of artery in case of stenting, to name a few.

As further seen, a particular type of decision node 206, for the treatment, is located one layer before the output layer 208. The last layer is the expected survival rates, in a form of KM curves 207, of the competing approaches 208 (e.g., of possible treatments from which to select). A graphical indication 210 marks, per branch of the survival tree graph, the best suited treatment for the physician to select.

### SURVIVAL DECISION TREE GRAPHS FOR PERSONALIZED CARDIAC ABLATION TREATMENT PLANNING

Regarding AF, the time origin discussed above can correspond to an invasive treatment of AF. The analysis of time-related events is unique, since the expected event - the recurrence of AF after a successful treatment - does not occur in most individuals. In the example of AF, KM curves describe the rate of treatment failure for AF over time.

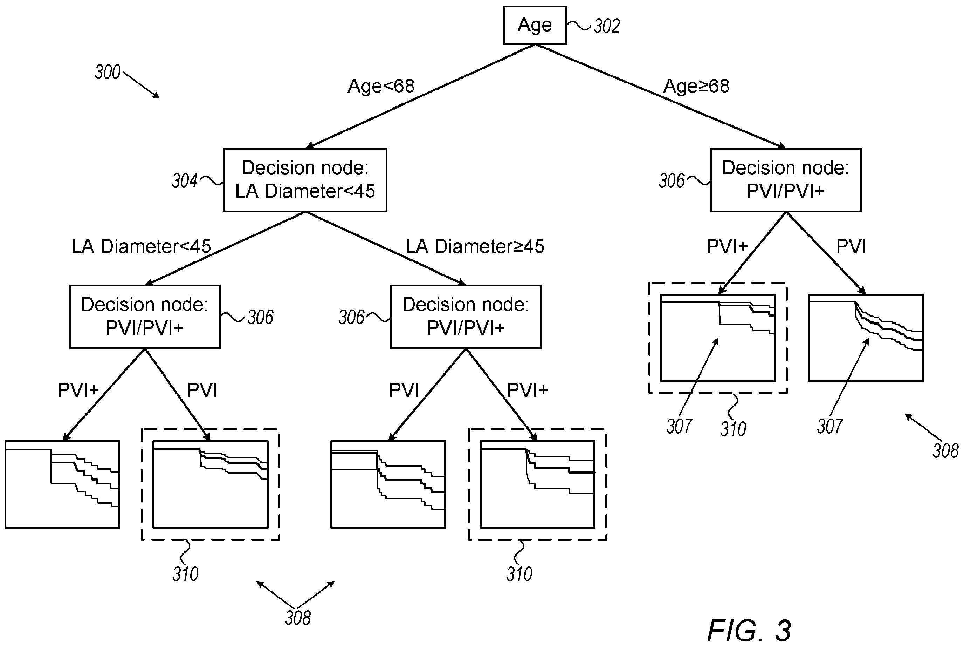

Fig. 3 illustrates a survival tree graph 300, generated by a genetic algorithm using Kaplan-Meier (KM) survival curves, to select an optimal ablation treatment for a given patient, according to an embodiment of the present invention.

The concluded strategy of the decision tree of Fig. 3 is:

**Table 1**

| **Group (decision node)** | **Recommended Strategy** |
|---|---|
| Age ≥ 68 | PVI plus |
| Age < 68, LA Diameter < 45 mm | PVI |
| Age < 68, LA Diameter ≥ 45 mm | PVI plus |

As seen, survival tree graph 300 comprises a root decision node 302. Tree graph 300 further comprises decision nodes 304 at different layers of the graph. Decision node 302 is based on age. Nodes 304 are based on age and on diameter of the left atrium (LA) as obtained by a diagnostic session (e.g., mapping and or imaging).

As further seen, a particular type of decision node 306, which is on the treatment, is located one layer before the output layer 308. The last layer is the expected survival rates, in four of KM curves 307, of the competing approaches 308 (e.g., of possible ablation treatments from which to select) . A graphical indication 310 marks, for the physician, the best-suited selection per branch of the survival tree graph.

Survival tree graph 300 can be stored in a memory stick or on a network drive, and provided to users of system 20 of Fig. 1, or to users of other systems.

The example illustration shown in Fig. 3 is chosen purely for the sake of conceptual clarity. Parameters that can be included in the creation of the a survival tree are numerous, preferably predictors of successful ablation approach, including the three column list:

**Table 2**

| Gender | Left ventricular ejection fraction < 50 | Sinus rhythm immediately before procedure |
|---|---|---|
| Ablation approach (PVI vs PVI plus) | Average length of persistent AF episodes | Structural heart disease |
| Other arrhythmia besides AF | Past successful cardioversion | Atrial flutter or tachycardia |
| Ventricular tachycardia | Previous ablation procedure | Age |
| Obstructive sleep apnea | LA diameter | |
| Hypertension | Pericardial effusion | |
| Diabetes | AAD Class I previous use | |
| Stroke | AAD Class III previous use | |
| Vascular disease | AAD Class V previous use | |
| Months since AF diagnosis | Number of past cardioversions | |

In addition to the above, parameters taken out of the atria mapping results can be included for the creation of the decision tree. These parameters can include:
- Percentage of low voltage zone area out of the atrium areas
- Number of focal triggers found by CARTOFINDER
- Number of non-PV triggers spotted by cycle length mapping, etc.
- Percentage area covered by fractionations out of the atrium area

### A METHOD OF GENERATING AND USING SURVIVAL DECISION TREE GRAPH FOR PERSONALIZED CARDIAC ABALATION TREATMENT PLANNING

Fig. 4 is a flow chart of a method for generating and using the genetic algorithm of Fig. 2 to select the optimal treatment procedure, according to an embodiment of the present invention. The algorithm, according to the presented embodiment, is divided into two parts: tree graph preparation 401 and tree graph use 402.

The algorithm carries out a process that begins with processor 222 receiving (e.g., uploading from memory 224) a medical database of patients that comprises survival rates, at a database receiving step 411. The database further comprises information such as appearing in Table 2.

Next, processor 222 generates a survival tree graph, such as tree graph 200, at a survival tree graph generation step 413. As described above, in each end branching of the graph, the graph provides maximal difference between two or more clinical treatment procedures among the different possible clinical treatment procedures.

Finally, for the part of tree graph preparation 401, the processor stores the generated survival tree graph in memory 224 and/or in non-transitory computer-readable medium, at a tree graph storage step 415.

At the beginning of the part of tree graph preparation using 402, a processor such as processor 41 of system 21 uploads the survival tree from, for example, non-transitory computer-readable memory medium, at a survival tree graph uploading step 420.

The processor next receives new patient medical data comprising, for example, results of an EP mapping procedure, at a new patient data receiving step 422.

Applying the survival tree graph to patient medical information, physician 30 selects an optimal clinical procedure (e.g., one of aforementioned ablation options) for the new patient, step 424. The optimum is guided by best expected outcome such as best MK curve for the new patient medical profile.

Although the embodiments described herein mainly address cardiac ablation, the methods and systems described herein can also be used in any medical field that involves a clinical decision between possible treatments.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A method, comprising:
receiving a data set comprising medical information of respective patients, respective types of a clinical procedure performed on the patients, and respective survival rates of the patients;
generating a survival tree graph by maximizing a cost function of differences in the survival rates between the types of the clinical treatment procedure; and
selecting a type of the clinical procedure for a given patient, based on the survival tree graph.

2. The method according to claim 1, wherein generating the survival tree graph comprises applying a genetic algorithm to the data set, optionally wherein applying the genetic algorithm comprises selecting decision nodes and cutoff values that maximize homogeneity in each split in the survival tree graph.

3. The method according to claim 1, wherein generating the survival tree graph further comprises fixing one or more layers of the survival tree graph.

4. The method according to claim 3, wherein fixing the layers comprises fixing a root of the survival tree graph, optionally wherein fixing the root of the survival tree graph comprises presetting the root to be one of an age and a gender of a patient.

5. The method according to claim 3, wherein fixing the layers comprises presetting a layer that precedes an output layer of the survival tree graph.

6. The method according to claim 5, wherein presetting the layer that precedes the output layer comprises presetting the layer to indicate a treatment approach.

7. A system, comprising:
an interface configured for receiving a data set comprising medical information of respective patients, respective types of a clinical procedure performed on the patients, and respective survival rates of the patients; and
a processor, which is configured to:
generate a survival tree graph by maximizing a cost function of differences in the survival rates between the types of the clinical treatment procedure; and
select a type of the clinical procedure for a given patient, based on the survival tree graph.

8. The method according to claim 1 or the system according to claim 7, wherein the differences in the survival rates comprises differences between Kaplan-Meier (KM) curves.

9. The system according to claim 7, wherein the processor is configured to generate the survival tree graph by applying a genetic algorithm to the data set, optionally wherein the processor is configured to apply the genetic algorithm by selecting decision nodes and cutoff values that maximize homogeneity in each split in the survival tree graph.

10. The system according to claim 7, wherein the processor is further configured to generate the survival tree graph by fixing one or more layers of the survival tree graph.

11. The system according to claim 10, wherein the processor is configured to fix the one or more layers by fixing a root of the survival tree graph, optionally wherein the processor is configured to fix the one or more layers by presetting the root to be one of an age and a gender of a patient.

12. The system according to claim 10, wherein the processor is configured to fix the one or more layers by presetting a layer that precedes an output layer of the survival tree graph.

13. The system according to claim 12, wherein the processor is configured to preset the layer that precedes the output layer by presetting the layer to indicate a treatment approach.

14. The method according to claim 6 or the system according to claim 13, wherein the treatment approach comprises pulmonary vein isolation.

15. The method according to claim 1 or the system according to claim 7, wherein the types of the clinical procedure are one of different cardiac ablation treatments of cardiac arrhythmia, different treatments of cancer, and different of treatments of brain stroke.

16. A computer software product, the product comprising a tangible non-transitory computer-readable medium in which program instructions are stored, which instructions, when read by a processor, cause the processor to:
generate a survival tree graph by maximizing a cost function of differences in the survival rates between the types of the clinical treatment procedure; and
select a type of the clinical procedure for a given patient, based on the survival tree graph.
